# EUROPEAN PATENT APPLICATION

(11) **EP 3 311 821 A1**
(43) Date of publication of application: **25.04.2018**
(21) Application number: 17185440.9
(22) Date of filing: 09.08.2017
(51) Int. Cl.: A61K 31/685, A61P 13/08, A61K 45/06, A61K 36/889, A61K 36/42

(54) **THE USE OF PHOSPHATIDYLSERINE FOR THE PREVENTION AND TREATMENT OF PROSTATE DISORDERS, AND THE CORRESPONDING PHARMACEUTICAL AND NUTRACEUTICAL COMPOSITIONS**

(30) Priority: 19.10.2016 IT 201600105201
(71) Applicant: Nutrilinea S.r.l., 21013 Gallarate (VA) (IT)
(72) Inventor: CORCIULO, Stefano, 21013 GALLARATE (VA) (IT); CALETTI, Rossella, 21013 GALLARATE (VA) (IT)
(74) Representative: Benedetto, Marco

(57) **Abstract**

The present invention relates to the use of phosphatidylserine for the prevention and treatment of prostate disorders, and the corresponding compositions.

## Description

The present invention relates to the use of phosphatidylserine for the prevention and treatment of prostate disorders, and the corresponding pharmaceutical and nutraceutical compositions.

### Technical background

The prostate is a gland present only in males, which is located immediately beneath the vesical trigone. Its function is to secrete a milky fluid containing citrate ions, calcium ions, phosphate ions and profibrinolysin. During sperm emission, the prostate gland contracts rhythmically in time with the contractions of the vas deferens and releases its secretion, which thus enriches the semen. The function of the prostate secretion appears to be to basify the vaginal environment. Said environment has a physiological pH of about 3.5-4.0, a value too low to ensure efficient sperm motility, which requires a pH of about 6.0-6.5.

Unfortunately, the prostate is often affected by pathological processes of various kinds and degrees of severity. Mild prostatitis (prostate inflammation with or without infection) is fairly common around the age of 45 years. After the age of 50, over 50% of men exhibit benign prostatic hyperplasia, involving a slow but progressive enlargement of the fibromuscular and epithelial structures of the gland. This phenomenon leads to obstruction of the urethra, urine retention and kidney damage, with consequent urinary disorders (frequent, difficult urination, painful perineal tension, incomplete bladder voiding, and reduced volume and flow of urine excreted). This disorder can be so serious as to require surgery.

Although the disorder is widespread, its etiology is still not fully understood. A variety of factors seem able to play a major part in the onset of prostatic hyperplasia: sex hormones, stromo-epithelial interactions, growth factors, insulin and prolactin.

An important part is certainly played by sex hormones, especially the male sex hormones. Testosterone, in particular, after diffusion through the plasma membrane, is converted by the action of 5-alpha reductase to di-hydrotestosterone (DHT). The latter, after binding to the androgen receptor, promotes protein synthesis, leading to cell growth. In this respect, abnormal hyperactivity of 5-alpha reductase is associated with hyperplasia of the gland. In fact, patients with prostatic hyperplasia exhibit DHT levels 4-6 times higher than normal.

Cholesterol, like its 5-alpha and 6-alpha epoxy-cholesterol metabolites, appears to play a significant part in the etiopathogenesis of prostate disease. Cholesterol and the esters thereof are involved in the metabolism of androgens, and are therefore responsible for increasing the number of androgen receptors.

The role of 5-alpha reductase and prostate tissue inflammation can be counteracted with medicaments such as finasteride, obtaining good results in the prevention and treatment of the symptoms associated with prostatic hyperplasia. However, the side effects of said medicaments are not well tolerated. They include iatrogenic impotence, which affects between 4 and 8% of users.

In the case of benign prostatic hypertrophy, pharmacological treatment is always essential to prevent the slow but inexorable progress of the symptoms and prevent complications, including acute urine retention.

The classes of medicaments mainly used to treat benign prostatic hypertrophy are:
- antiandrogens, in particular dutasteride and finasteride; specific 5-alpha reductase inhibitors; able to reduce the size of the prostate gland and the symptoms of obstruction, and improve the urinary function;
- adrenergic drugs, especially alpha-blockers; widely used in the treatment of prostatic hypertrophy, they act by relaxing the smooth muscles, inducing a significant increase in urine flow, and improving the symptoms of obstruction, which can be toxic at renal level.

Phosphatidylserine (PS) belongs to the class of phospholipids, ie. molecules that make up the cell membranes. In addition to PS, which is present to a low extent in animal tissues, phospholipids include phosphatidic acid, phosphatidylcholine, phosphatidylethanolamine, phosphatidylglycerophosphate, diphosphatidylglycerols and phosphatidylinositol.

It has long been known that PS has favourable effects on the memory and cognitive faculties. A number of studies confirm the properties of PS in terms of reinstatement of the memory capacity, especially if associated with aging. PS is also used as an ergogenic aid in sport, as an antidepressant in neurology, as a galactogen for hypogalactia, and as a sedative for anxiety.

### Description of the invention

It has now been discovered that phosphatidylserine (PS) performs a strong anti-prostatitis action, demonstrated by a reduction in prostatic hyperplasia.

The present invention therefore relates to the use of phosphatidylserine for the prevention and treatment of prostate disorders in humans, and pharmaceutical and nutraceutical compositions containing phosphatidylserine for the prevention and treatment of prostate disorders in humans.

According to a preferred aspect of the invention, the prostate disorder is benign prostatic hyperplasia.

According to a preferred aspect of the invention, the compositions will contain phosphatidylserine in quantities such as to ensure the administration of daily doses ranging between 5 and 500 mg, preferably between 5 and 250 mg, and more preferably between 5 and 100 mg, optionally divided between two or three daily administrations.

According to a preferred aspect of the invention, the compositions may contain other ingredients with complementary or useful activities for the intended use. Examples of said ingredients include vitamins (A, B group, C, D, E, F and K), mineral salts (calcium, zinc, iron, copper, magnesium, manganese, selenium and potassium), and phytotherapeutic medicaments such as serenoa (*Serenoa repents*)*,* pygeum (*Pygeum africanum*), nettle (*Urtica dioica*), beta-sitosterol (*Hypoxis rooperi*) and pumpkin (*Cucurbita pepo*).

According to a further preferred aspect of the invention, the compositions may also contain herbal derivatives used to reduce the possible transformation and progression from benign to malignant disease, such as lycopene from tomato skins (or other sources) and soybean *(Glycine max*) isoflavones.

According to a further preferred aspect of the invention, the compositions will contain phosphatidylserine in the form of phytosomal carriers according to the technique described in EP 209038.

The doses of the other ingredients, if any, can also vary within wide ranges, corresponding to those already used for the same ingredients for different indications.

According to a preferred aspect of the invention, the compositions will contain serenoa oil in quantities ranging between 10 and 500 mg, and in particular between 40 and 320 mg.

Similarly, according to a preferred aspect of the invention, the compositions will contain pumpkin in quantities ranging between 10 and 500 mg, and in particular between 40 and 320 mg.

According to a preferred aspect of the invention, the compositions will contain pygeum derivatives in quantities ranging between 10 and 200 mg, and more typically between 50 and 100 mg.

In the same way, according to a preferred aspect of the invention, the compositions will contain beta-sitosterol in quantities ranging between 5 and 600 mg, and more typically between 20 and 100 mg.

### TRIAL

The trial was conducted in accordance with the murine model described in Prostate 2003;54(1):17-24 (Takao T. *et al.* Stromal/epithelial interactions of murine prostatic cell lines in vivo: a model for benign prostatic hyperplasia and the effect of doxazosin on tissue size). It was demonstrated that the use of PS (0.1 ml/day), administered orally, reduced the subcutaneous growth of the line implanted, measured on the basis of the diameters viewable from the exterior, by about 35%. Moreover, examination of a histological section demonstrated a 20% reduction in hyperplastic phenomena measured on the basis of Ki67 expression. In detail, the cell line implantation in the Balb/c mouse aged about 8 weeks and weighing 32+/-6 grams induced an evident proliferative response, with a measurable subcutaneous mass (2.2 +/-0.4 cm) in the next 90 days. Treatment with serenoa oil (0.2 ml/day; p.o.) produced a reduction (albeit not a significant one) of about 5% in the mean of the diameters, and about a 2.8% reduction in Ki67 expression. Comparable results were obtained after treatment with pumpkin oil (0.2 ml/day; s.c.). Conversely, treatment with pygeum extract (2.5 mg/day; p.o.) or nettle extract (4.0 mg/day; p.o.) did not produce appreciably different results from those obtained in the control group. However, combined administration of PS with serenoa and pumpkin oil, at doses exactly half of those indicated above, reduced hyperplasia, measured as the mean of the diameters, by 45% compared with the control group (p<0.05). Conversely, Ki67 expression was 28% lower (p<0.05).

### FORMULATION ASPECTS

The compositions according to the present invention can be formulated suitably for oral administration, and will be prepared by conventional methods well known in pharmaceutical technology, such as those described in Remington's Pharmaceutical Handbook, Mack Publishing Co., N.Y., USA. Examples of said formulations are tablets, capsules, oral preparations, powders, granules, pastilles, reconstitutable powders, solutions or suspensions, generally presented in unit dose form and containing conventional excipients such as binders, fillers, diluents, compression agents, lubricants, disintegrants, colorants, flavouring agents, wetting agents, anti-caking agents, etc., which are acceptable for their final use.

Some examples of formulations are set out below.

### Examples of formulations

### Example 1

Soft gelatin capsules containing:
1) Phosphatidylserine 5 mg/dose
2) standardised oil of *Serenoa repens* 320 mg/dose
3) standardised oil of *Cucurbita pepo* 40 mg/dose

### Example 2

Soft gelatin capsules containing:
1) Phosphatidylserine 5 mg/dose
2) standardised oil of *Serenoa repens* 160 mg/dose
3) standardised oil of *Cucurbita pepo* 80 mg/dose

### Example 3

Sachets containing (phases in oil on adsorbent phase):
1) Phosphatidylserine 5 mg/dose
2) standardised oil of *Serenoa repens* 160 mg/dose
3) standardised oil of *Cucurbita pepo* 160 mg/dose
4) vitamin C 100% NRV
5) vitamin E 100% NRV
6) copper oxide 100% NRV
7) zinc oxide 100% NRV
NRV: Nutrient Reference Values

### Example 4

Sachets containing (phases in oil on adsorbent phase):
1) Phosphatidylserine 5 mg/dose
2) standardised oil of *Serenoa repens* 160 mg/dose
3) standardised oil of *Cucurbita pepo* 160 mg/dose
4) vitamin C 100% NRV
5) vitamin E 100% NRV
6) copper oxide 100% NRV
7) zinc oxide 100% NRV
8) lycopene 6 mg/dose
9) isoflavones (calculated as aglycons) 40 mg/dose
NRV: Nutrient Reference Values

## Claims

1. Phosphatidylserine for use in the prevention and treatment of prostate disorders.

2. Phosphatidyl serine for use according to claim 1, wherein the prostate disorder is benign prostatic hyperplasy.

3. Pharmaceutical and nutraceutical compositions comprising phosphatidylserine for use in the prevention and treatment of prostate disorders.

4. Pharmaceutical and nutraceutical compositions for use according to claim 3, further comprising other ingredients with complementary activity selected from vitamins (A, group B, C, D, E, F and K), mineral salts (calcium, zinc, iron, copper, magnesium, manganese, selenium, potassium) and phytotherapeutics such as serenoa (*Serenoa repens*), pigeo *(Pygeum africanum*), ortica (*Urtica dioica*), beta-sitosterol (*Hypoxis rooperi*), cucurbita (*Cucurbita pepo*), lycopene from tomato peel (or any other source) and soy bean (Glycine max) isoflavones.
